# FASCICULE DE BREVET EUROPEEN

(11) **EP 0 951 290 B1**
(45) Date de publication et mention de la délivrance du brevet: **13.03.2002**
(21) Numéro de dépôt: 97952992.2
(22) Date de dépôt: 23.12.1997
(51) Int. Cl.: A61K 35/74, A23L 1/03, A23L 1/308, A23C 19/032

(54) **COMPOSITION ABSORBABLE RENFERMANT DES BACTERIES PROPIONIQUES SUSCEPTIBLE DE DEGAGER DU MONOXYDE D'AZOTE DANS LE TUBE DIGESTIF HUMAIN OU ANIMAL**
ABSORBIERBARE ZUSAMMENSETZUNG ENTHALTEND PROPIONÄURE BAKTERIEN DIE FÄHIG SIND, STICKSTOFFOXID IN DEM MENSCHLICHEN ODER TIERISCHEN VERDAUUNGSTRAKT ZU PRODUZIEREN
ABSORBABLE COMPOSITION CONTAINING PROPIONIC BACTERIA CAPABLE OF RELEASING NITRIC OXIDE IN THE HUMAN OR ANIMAL ALIMENTARY CANAL

(30) Priorité: 24.12.1996 FR 9615977; 28.01.1997 FR 9700885
(43) Date de publication de la demande: 27.10.1999
(73) Titulaire: Laboratoires Standa S.A., 14050 Caen Cédex (FR)
(72) Inventeur: ROUSSEL, Edmond, Daniel, F-14210 Avenay (FR); LEGRAND, Charles, Gabriel, F-14000 Caen (FR); LEGRAND, Marc, Henri, F-14000 Caen (FR); ROLAND, Nathalie, F-35000 Rennes (FR)
(74) Mandataire: Cabinet HERRBURGER
(86) Numéro de dépôt international: FR9702399
(87) Numéro de publication internationale: WO9827991

(56) Documents cités:
- EP-A- 0 071 858
- DATABASE MEDLINE FILE SERVER STN KARLSRUHE ABR G 93363965, PAWLIK ET AL: "MICROCIRCULATORY AND MOTOR EFFECTS OF ENDOGENOUS NITRIC OXIDE IN THE RAT GUT" XP002039361 & JOURNAL OF PHYSIOLOGY AND PHARMACOLOGY, vol. 44, no. 2, juin 1993, pages 139-146,
- DATABASE MEDLINE FILE SERVER STN KARLSRUHE ABR G 93146090, CALIGNANO ET AL: "INVOLVEMENT OF ENDOGENOUS NITRIC OXIDE IN THE REGULATION OF RAT INTESTINAL MOTILITY IN VIVO" XP002039362 & EUROPEAN JOURNAL OF PHARMACOLOGY, vol. 229, no. 2-3, 15 décembre 1992, pages 273-276,

## Description

La présente invention concerne une composition d'alimentation courante ou une composition diététique ou médicamenteuse absorbable renfermant des bactéries propioniques susceptibles de dégager des quantités physiologiquement significatives de monoxyde d'azote dans le tube digestif humain ou animal.

Pendant de nombreuses décennies, on a totalement ignoré que le monoxyde d'azote constitue l'un des éléments nécessaires à la vie et à son maintien ; par suite jusqu'à ces quatre ou cinq dernières années, les chercheurs ne se sont pas penchés sur les bienfaits associés à la présence de cet oxyde, que ce soit en médecine, en nutrition ou en physiologie.

Ce n'est que tout dernièrement que l'on a attribué au monoxyde d'azote un nombre impressionnant de fonctions physiologiques et que l'on a émis l'hypothèse que ce gaz pouvait être impliqué au premier chef dans des fonctions aussi diverses que le contrôle de la pression artérielle, la fonction cytotoxique non spécifique des macrophages, l'agrégation plaquettaire et la neurotransmission ou encore le contrôle de la motricité du tube digestif.

A partir de cette supposition, les recherches portant sur le monoxyde d'azote se sont multipliées et l'importance de ce gaz a pu être confirmée.

Il est connu que le monoxyde d'azote, qui est un gaz très instable (demi-vie inférieure à 5 secondes dans les systèmes biologiques), est produit par biosynthèse au sein de l'organisme humain ou animal à partir de la L-arginine par un groupe d'enzymes dénommées NO-synthases (NOS) dont il existe deux types principaux, à savoir d'une part les NOS constitutives qui sont exprimées notamment dans les cellules endothéliales, les plaquettes sanguines et les neurones et, d'autre part, les NOS inductibles qui sont exprimées principalement par certaines cellules du système immunitaire (macrophages et polynucléaires notamment) par le muscle lisse vasculaire et les cellules endothéliales.

Il est à noter que la production de NO par les NOS inductibles est, de plusieurs ordres de grandeurs, supérieure à la production de NO par les NOS constitutives, mais que dans tous les cas, cette production demeure relativement faible.

Or, et compte tenu du rôle bénéfique susmentionné du monoxyde d'azote, il serait souhaitable de pouvoir augmenter cette production en particulier en utilisant la voie naturelle du métabolisme alimentaire.

On n'a cependant jusqu'à présent jamais proposé de moyens permettant de parvenir à ce résultat.

L'objet de l'invention est de combler cette lacune.

Conformément à l'invention, on a pu parvenir au but recherché en constatant que, de manière surprenante, un type particulier de bactéries, les bactéries propioniques, sont susceptibles de produire du monoxyde d'azote, et que parmi ces bactéries, certaines espèces et certaines souches parmi ces espèces en produisent de grandes quantités.

Il s'agit là de bactéries propioniques qui, bien que n'appartenant pas au groupe des bactéries lactiques ou bifidobactéries classiquement introduites dans l'organisme par le biais de desserts lactés ou autres produits laitiers fermentés, sont néanmoins présentes en alimentation humaine depuis des siècles : ce sont en effet elles qui permettent l'obtention des trous lors de la fabrication du fromage dénommé « emmental » qui en fin d'affinage renferme environ 10⁹ cellules/g de bactéries propioniques.

Il est à noter que la fermentation de ces bactéries produit entre autre de l'acide propionique, de l'acide acétique et du dioxyde de carbone.

La constatation susmentionnée est d'autant plus surprenante que l'on a pu vérifier que des bactéries lactiques, des bifidobactéries et/ou des levures, utilisées couramment dans le domaine agro-alimentaire, ne produisent pas de monoxyde d'azote.

L'invention se rapporte, en conséquence, à l'utilisation de bactéries propioniques pour l'obtention d'une composition d'alimentation courante ou d'une composition diététique ou médicamenteuse absorbable susceptible de dégager des quantités physiologiquement significatives de monoxyde d'azote dans le tube digestif humain ou animal.

Conformément à l'invention, cette composition peut être constituée par une préparation élaborée et/ou présentée sous forme liquide (en particulier d'un liquide fermenté), sous forme déshydratée ou d'humidité intermédiaire.

Plus précisément, il est à noter que, sans pour cela sortir du cadre de l'invention, la composition peut se présenter :
- soit sous forme d'une préparation spécifique se justifiant par sa seule finalité physiologique, à savoir l'ingestion de bactéries propioniques susceptibles de dégager des quantités physiologiquement significatives de monoxyde d'azote,
- soit sous forme d'une préparation alimentaire élaborée ayant parallèlement une finalité autre plus strictement énergétique ou fonctionnelle ; dans ce dernier cas, les bactéries propioniques peuvent être ajoutées ou incorporées dans les aliments eux-mêmes notamment dans des fromages, dans des fibres alimentaires telles que des flocons de céréales, ou encore dans des laits fermentés, crèmes desserts, gâteaux et/ou boissons bienfaisantes, etc..

Conformément à l'invention, les bactéries propioniques peuvent être introduites sous forme de biomasse ou sous la forme d'un levain susceptible de se multiplier in situ.

Lorsqu'elle est déshydratée, la composition se présente, avantageusement, sous forme de fractions individuelles renfermant la dose de bactéries devant être absorbée régulièrement.

Ces fractions peuvent être ingérées directement ou être préalablement diluées dans un liquide ; elles peuvent être conditionnées sous une forme permettant de faciliter l'absorption : comprimés, sachets de poudre granulée, liquide, ...

On a vérifié que de telles préparations déshydratées concentrées de bactéries propioniques conservées une année à +4°C voient leur concentration baisser de moins de un Log.

L'expérience a prouvé que des gélules gastrorésistantes ou non correspondent à un type de conditionnement particulièrement avantageux.

Selon une autre caractéristique de l'invention, chaque fraction individuelle renferme un grand nombre de bactéries, de préférence plus de 10⁹ bactéries.

Diverses expérimentations (résumées ci-dessous) ont permis de vérifier l'aptitude toute particulière de différentes souches de bactéries propioniques à produire du NO au cours de leur culture, d'abord indirectement à partir de la mesure des ions nitrites NO₂ puis directement par analyse en spectrométrie de masse en milieu anaérobie.

Au cours de ces expérimentation, on a, dans un premier temps, étudié la concentration en nitrites dans des milieux riches en arginine et pauvres en nitrates (50 µM) et on s'est rendu compte que l'arginine n'est pas un élément déterminant dans la production de NO observée.

Dans un second temps, on a expérimenté des milieux supplémentés en nitrates. Les résultats obtenus dans ce dernier cas ont révélé le caractère nitrate dépendant de la production de monoxyde d'azote.

### 1 - Essais préliminaires comparatifs

Différentes souches bactériennes (inoculum yaourt, bifidobactéries, lactobacillus) ont été cultivées en présence d'un milieu lait reconstitué (100 ml) supplémenté par un extrait de levures (10 g/l) puis incubées à 37°C.

L'accumulation de nitrite a été mesurée au cours du temps.

Ces essais préliminaires ont été réalisés dans les conditions suivantes :
- incubation à 37°C pendant 0, 4, 7 ou 10 heures,
- 3 répétitions,
- dosage des nitrites par système Bran-Luebbe.

En raison de la nature des extraits à analyser, une étape de purification des échantillons a été successivement mise en oeuvre par une double centrifugation (2 x 10 min., 4°C, 15 000 rpm), suivie d'une ultrafiltration sur cartouche miniprep 10 (rétention des protéines de PM > 10kD) puis d'une purification partielle par passage de l'échantillon sur résine Waters C18 (55-105 µm).

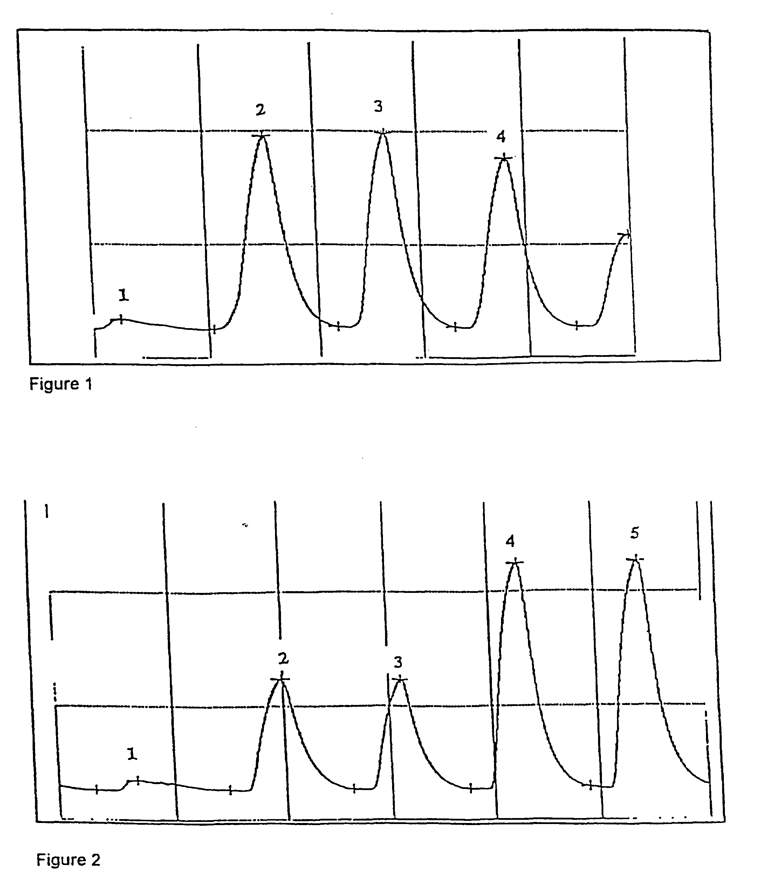

Cette méthode a, dans un premier temps, été testée sur des échantillons étalons de nitrite (Figure 1), puis sur des extraits de culture de *Lactobacillus* incubés 7 heures auxquels a été ajoutée ou non une quantité connue de nitrite (Figure 2).

La figure 1 représente les profils colorimétriques obtenus sur une chaîne d'analyse automatique Bran Luebbe :
(1) d'un milieu de culture de bifidobactéries après 10 heures d'incubation,
(2) d'une solution standard de nitrite,
(3) de cette même solution ultrafiltrée,
(4) de cette même solution ultrafiltrée et passée sur résine C18.

La figure 2 représente les profils colorimétriques obtenus sur une chaîne d'analyse automatique Bran Luebbe :
(1) d'un milieu de culture de *Lactobacillus* après 10 heures d'incubation à 37°C,
(2) (3) d'une solution standard contenant 410 µg de nitrite/l,
(4) (5) d'un milieu de culture de *Lactobacillus* après 10 heures d'incubation à 37°C auxquels a été ajoutée une quantité connue de nitrite afin d'obtenir une solution à 820 µg/l de nitrite.

Ces échantillons ont été purifiés par centrifugation ultrafiltration et passage sur résine C18 dans les conditions décrites précédemment.

Conformément à ces essais, aucune accumulation de nitrite n'a pu être détectée que ce soit à partir d'inoculum de yaourt, de bifidobactéries ou de *Lactobacillus*, ce quel que soit le temps d'incubation (0, 4, 7 ou 10 heures).

### 2 - Mise en évidence de l'accumulation de nitrite par des cultures de bactéries propioniques

On a préalablement recherché la présence éventuelle de nitrate ou de nitrite dans la préparation du milieu YEL par dosage colorimétrique (kit Boerhinger) : on a ainsi pu mettre en évidence la présence d'une quantité non négligeable de nitrate dans ce milieu (concentration de l'ordre de 50 à 100 µM) qui pourrait provenir de l'extrait de levure utilisé pour la fabrication de ce milieu ; en revanche, on a vérifié que le milieu YEL était totalement exempt de nitrite.

Des cultures de bactéries propioniques (1 g de lyophilisat pour 100 ml de milieu YEL) ont été testées.

Ces essais ont été mis en oeuvre dans les conditions suivantes :
- incubation à 30°C pendant 24, 48 ou 72 heures,
- 3 répétitions pour l'incubation de 24 heures,
- arrêt de l'incubation par ébullition,
- purification du produit par centrifugation et passage de l'extrait sur résine C18,
- dosage des nitrites dans le milieu par analyse sur système Bran-Luebbe.

On a dosé les nitrites accumulés par les bactéries propioniques afin d'établir une cinétique d'accumulation des nitrites en fonction du temps d'incubation des bactéries sur milieu YEL.

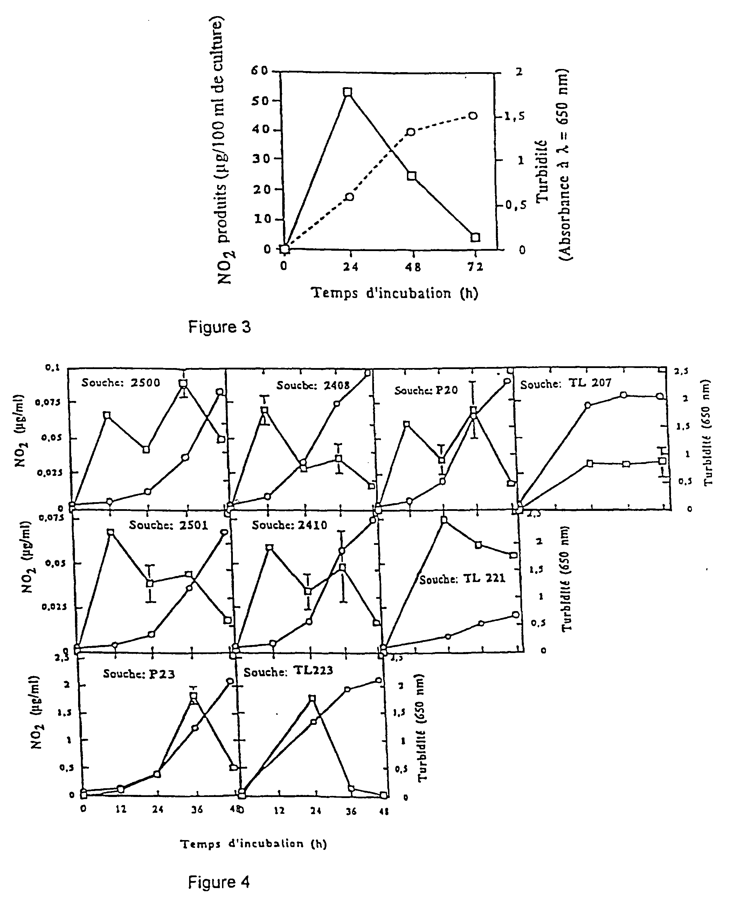

La figure 3 représente, d'une part, les variations de la quantité de nitrite produite (en µg/100 ml de culture) en fonction du temps d'incubation (en heures) (□) et d'autre part les variations de la turbidité (absorbance à λ = 650 nm) également en fonction du temps d'incubation (O).

Cette figure montre que la quantité de nitrite est maximale à 24 heures puis diminue ensuite de façon significative après 48 et 72 heures d'incubation.

On peut raisonnablement penser que cette chute résulte de la réduction du nitrite en NO, N₂O ou autres composés par la nitrite réductase.

Conformément à l'invention, on a pu prouver que l'accumulation de NO₂ dépend des espèces ou souches de bactéries propioniques mises en oeuvre.

Cette situation a été vérifiée par les essais résumés ci-dessous :

### 3 - Mise en évidence et comparaison des accumulations de nitrite dans le milieu de culture dans le cas de 9 souches de 4 espèces différentes de bactéries propioniques

Conformément à cet essai, on a étudié les souches P20, P23, 2408, 2410, 2500 et 2501 de l'espèce *P.freudenreichii* et les souches TL221, TL223 et TL207 appartenant respectivement aux espèces *P.thoenii, P.acidipropionici* et *P.jensenii.*

Il est à noter que les souches TL (technologie laitière) sont des souches appartenant à l'INRA-LRTL, tandis que la souche P23 (ou ITG23) a été enregistrée à la Collection Nationale des Cultures de Micro-organismes (CNCM) de l'Institut Pasteur sous le numéro I-1804 en date du 18.12.96.

Les différentes souches de bactéries propioniques (1 g de lyophilisat ou 5 ml de culture fraîche) ont été cultivées sur 100 ml de milieu YEL contenant environ 50 µM de nitrate, selon les conditions suivantes :
- incubation à 30°C pendant 12, 24, 36 ou 48 heures,
- 3 répétitions,
- arrêt de l'incubation par ébullition,
- purification du produit par centrifugation et passage de l'extrait sur résine C18,
- accumulation de nitrite dans le milieu, mesurée par analyse sur système Bran-Luebbe,
- estimation de la fermentation de chaque culture mesurée par lecture de l'absorbance à 650 nm.

Les résultats obtenus pour chaque souche sont rassemblés sur la figure 4.

Celle-ci représente, dans chaque cas, les variations de l'accumulation de nitrite (□) et de la turbidité du milieu de culture (O) en fonction de la durée d'incubation. Chaque valeur correspond à la moyenne ± l'écart type de la moyenne pour n = 3.

Il convient de noter que les échelles d'accumulation du nitrite sont 25 fois plus grandes dans le cas des souches P23 et TL223.

Ces résultats prouvent que la croissance bactérienne, estimée d'après l'évolution de la turbidité du milieu de culture, est proche pour l'ensemble des souches étudiées, atteignant environ 2 à 2,5 DO après 48 heures d'incubation, à l'exception de la souche TL221 pour laquelle la turbidité n'atteint que 0,6 DO après 2 jours.

En revanche, il existe des différences hautement significatives quant à l'accumulation de nitrite en fonction du temps.

En effet, les souches 2500, 2408, P20, 2501, 2410, TL207 et TL221 accumulent une quantité relativement peu importante de nitrite, le maximum (0,1 µg de NO₂⁻/ml) étant atteint respectivement après 36, 12, 36, 12, 12, 24 et 24 heures d'incubation.

A l'opposé, une accumulation beaucoup plus forte de nitrite a été obtenue avec des souches P23 et TL223 qui accumulent, au maximum, 1,8 µg de NO₂⁻/ml après, respectivement, 36 et 24 heures d'incubation.

Il est à noter que la souche de bactéries propioniques analysée dans le second essai susmentionné (figure 3) avait une position intermédiaire avec une accumulation maximale de NO₂⁻, d'environ 0,5 µg/ml.

Ces essais ont donc permis de constater qu'il existe des différences significatives entre les quantités de nitrite pouvant être produites par différentes souches de bactéries propioniques de quatre espèces différentes, ces différences étant indépendantes de la croissance de ces souches.

Ces résultats ont pu être confirmés par l'étude pour chaque souche de l'évolution de la concentration en nitrite du milieu de culture en fonction de sa turbidité et donc approximativement de la croissance bactérienne.

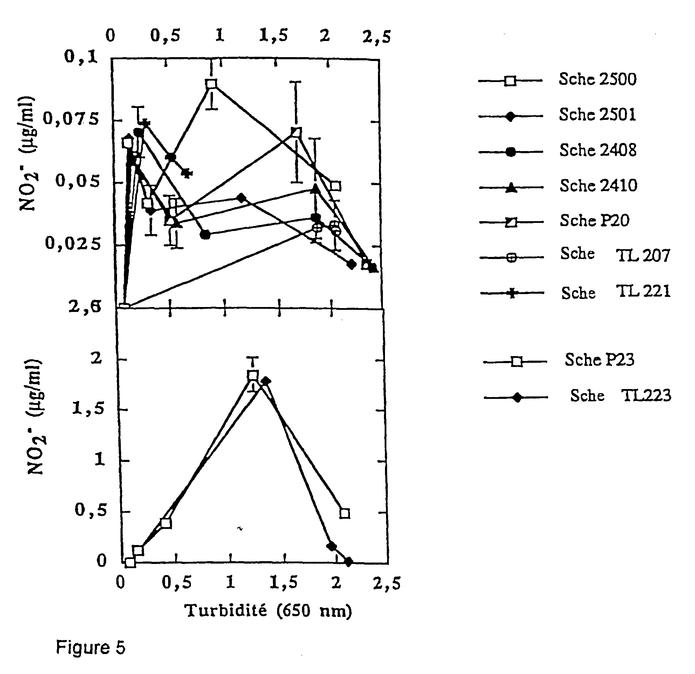

Les résultats de ces derniers essais sont rapportés sur la figure 5 sur laquelle chaque valeur correspond à la moyenne ± l'écart type de la moyenne pour n = 3.

Les essais susmentionnés ont été de nature à établir que, parmi les souches étudiées, la souche TL223 est la plus fortement accumulatrice de nitrites, ces nitrites disparaissant après 12 heures. Cette souche a donc été retenue dans le cadre d'essais complémentaires portant sur la mesure directe de la production de monoxyde d'azote par analyse en spectrométrie de masse en milieu anaérobie.

### 4 - Mesure préliminaire de la production de NO par la souche TL223 sous atmosphère d'hélium

Conformément à ces essais, les cultures ont été réalisées dans des tubes de 10 ml contenant 5 ml de milieu YEL contenant environ 50 µM de nitrate et 0,25 ml de culture fraîche de la souche TL223.

L'atmosphère des tubes a été immédiatement évacuée par un flux d'hélium (100 ml/min.) pendant 100 secondes.

L'accumulation de NO dans l'atmosphère des tubes a ensuite été mesurée au cours du temps dans les conditions suivantes :
- incubation à 30°C pendant 24, 48 ou 72 heures,
- 4 répétitions,
- mesure de l'accumulation de NO par analyse en spectrométrie de masse,
- estimation de la fermentation de chaque culture mesurée par lecture de l'absorbance à 650 nm.

Lors d'un essai préliminaire, le système de purification de gaz (Roboprep G+) - spectromètre de masse (Twenty-Twenty) a été calibré par des quantités croissantes de monoxyde d'azote.

Ce gaz a été généré à partir de NaNO₂ en présence d'une solution de KI et H₂SO₄.

L'identification et la quantification du monoxyde d'azote ont été réalisées par sa masse : 30 pour ¹⁴N¹⁶O et 31 pour ¹⁵N¹⁶O et ¹⁴N¹⁷O. Cette identification a ensuite été confirmée par la mesure du rapport isotopique : 31/30 = [¹⁵N¹⁶O + ¹⁴N¹⁷O]/¹⁴N¹⁶O. Il est à noter que le rapport isotopique théorique 31/30 du NO est de 0,00367 en l'absence de contamination par l'¹⁷O.

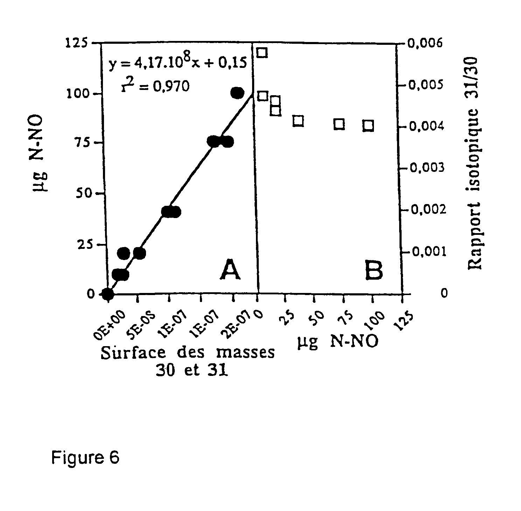

Les résultats de cet essai préliminaire sont rapportés sur la figure 6 sur laquelle la partie gauche (A) correspond à la courbe d'étalonnage du spectromètre de masse utilisé pour la quantification du monoxyde d'azote tandis que la partie droite (B) correspond à la mesure du rapport isotopique 31/30.

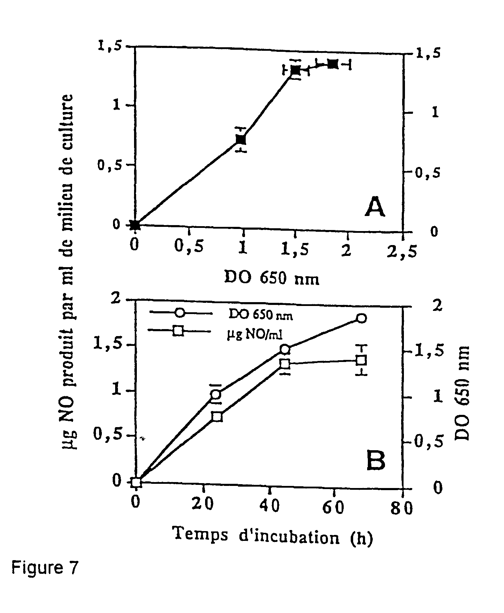

Les résultats proprement dits de cet essai sont rapportés sur la figure 7.

Plus précisément, la figure 7A représente les variations de l'accumulation de NO dans l'atmosphère des tubes en fonction de la turbidité du milieu tandis que la figure 7B représente les variations de cette accumulation en fonction du temps d'incubation.

Les barres verticales ou horizontales indiquent, lorsqu'elles sont plus larges que le symbole, l'écart type de la moyenne pour n = 4.

Une comparaison de la figure 7B (qui représente l'évolution au cours du temps de la turbidité du milieu de culture sous hélium) avec la figure 4 (qui représente cette même évolution à l'air) montre que la croissance de la souche TL223 n'est pas affectée significativement par une atmosphère constituée essentiellement d'hélium.

On a également pu établir que la vitesse d'accumulation du monoxyde d'azote dans l'atmosphère est constante durant environ les 45 premières heures d'incubation, puis s'infléchit ensuite (figure 7B), ce qui correspond à une turbidité proche de 1,5 DO (figure 7A).

Après environ 65 heures d'incubation (turbidité supérieure à 1,7 DO), environ 1,5 µg de NO sont accumulés dans l'atmosphère d'hélium pour 1 ml du milieu de culture.

L'ordre de grandeur obtenu est compatible avec les teneurs en nitrite mesurées dans le milieu pour des cultures au contact de l'air.

Dans ce dernier cas, on avait en effet pu constater (figure 5) que la souche TL223 accumulait au maximum 1,8 µg de NO₂⁻/ml pour une turbidité de 1,5 DO, ce qui correspond à environ 1,2 µg de NO/ml.

A partir de cette mesure directe préliminaire de la production de NO par la souche TL223, on a cherché, conformément à l'invention, à confirmer la voie de synthèse de ce monoxyde d'azote, et on a eu à cet effet l'idée de rechercher si celle-ci est stimulée par un apport de nitrite ou de nitrate.

Une telle stimulation a pu être vérifiée grâce aux essais résumés ci-dessous :

### 5 - Etude de la stimulation de la production de NO par apport de nitrite ou de nitrate

Dans le but de rechercher si la production de NO par les bactéries propioniques est possible à partir de NO₂⁻ ou de NO₃⁻, on a recherché si l'on observe une augmentation de la production de NO par la souche TL223 lorsqu'elle est en présence de 1 mM de KNO₂ ou de KNO₃ (avec et sans marquage isotopique au ¹⁵N.

Cette expérimentation a été effectuée dans les conditions suivantes :
- la souche TL223 a été ensemencée à 1 % dans un milieu YEL seul (témoin) ou avec addition de 1 mM de KNO₂, de KNO₃ ou de K¹⁵NO₃ avec un marquage isotopique à ¹⁵N à 50%),
- incubation à 30°C pendant 24, 48 et 72 heures,
- 3 répétitions par point et par traitement,
- accumulation du NO et détermination du rapport isotopique par spectrométrie de masse,
- estimation de la fermentation de chaque culture (turbidité) mesurée par lecture de l'absorbance à 650 nm,
- dosage du nitrate et du nitrite après récupération des milieux bactériens, centrifugation et mesure par kit Boerhinger.

On a également analysé l'accumulation de NO en fonction de la concentration en NO₃⁻ (100, 150, 350, 650 et 1050 µM) ou en NO₂⁻ (50, 100, 400, 800, 1000 µM) après une incubation de 72 heures à 30°C (TL223 ensemencée à 1 % dans un milieu YEL).

Les échantillons à analyser ont été distribués dans des tubes d'analyse de NO dont l'atmosphère a été immédiatement évacuée par un flux d'hélium (flush à l'hélium) pendant 150 s afin d'obtenir des conditions anaérobies strictes.

L'effet du flush à l'hélium sur une éventuelle détection de NO par le spectromètre de masse a été testé sur un milieu YEL stérile : ce milieu YEL, préalablement flushé à l'hélium, a été incubé à 30°C pendant 72 heures. Après 0, 24, 48 et 72 heures d'incubation (3 répétitions) on n'a détecté aucune production de NO et les valeurs de turbidité sont restées nulles.

On a ainsi pu vérifier la qualité du flush à l'hélium, l'absence de toute contamination bactérienne et l'absence d'interaction entre le flush à l'hélium et la mesure de NO par spectrométrie de masse.

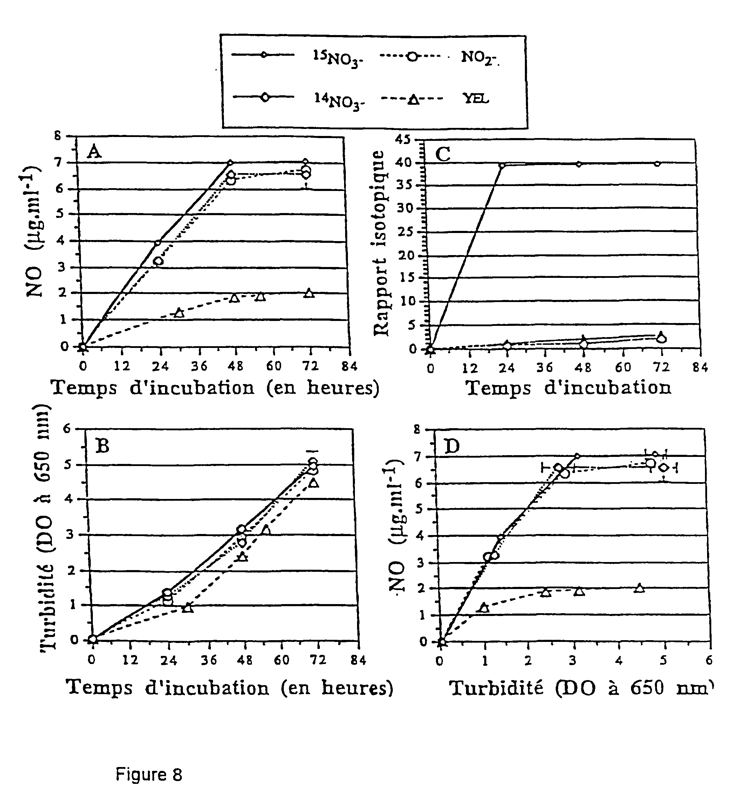

Les essais susmentionnés ont permis d'obtenir les résultats rapportés sur la figure 8, en ce qui concerne la cinétique d'accumulation du NO en présence de nitrite ou de nitrate.

Plus précisément :
- la figure 8A représente les variations de l'accumulation de NO en fonction du temps,
- la figure 8B représente les variations de la turbidité en fonction du temps,
- la figure 8C représente les variations du rapport isotopique [masse 31/(masses 30+31)] en fonction du temps,
- la figure 8D représente les variations de la production de NO en fonction de la turbidité.

Chacune de ces figures concerne la souche TL223 cultivée sur un milieu YEL seul, en présence de 1 mM de nitrate (¹⁵NO₃⁻ - marqué à 50 % et ¹⁴NO₃⁻) et en présence de 1 mM de nitrite.

Les barres verticales représentent ± l'écart type de la moyenne pour n = 3 lorsqu'elles sont plus grandes que le symbole.

Ces figures prouvent que l'accumulation de NO chez TL223 cultivé sur 1 mM de KNO₃ (marqué à l'azote 15 ou non marqué) ou sur 1 mM de KNO₂ est proche de 7 µg de NO/ml après 48 heures d'incubation à 30°C ; cette valeur est 3,5 fois supérieure à la production de NO dans le cas d'un milieu YEL non supplémenté en nitrate ou en nitrite (figure 8A). Ces différences ne sont pas dues à des variations de croissance engendrées par la composition du milieu car la turbidité en fin de croissance, est du même ordre de grandeur sur un milieu YEL seul (4,5 unités DO après 72 heures) et sur un même milieu additionné en nitrate ou en nitrite (environ 5 unités DO après 72 heures - figures 8B et 8D).

La figure 8C révèle que l'apport de K¹⁵NO₃ marqué à 50 % permet d'obtenir du NO avec un marquage à environ 40 % après 48 heures d'incubation : l'azote de masse 15 apporté sous forme de nitrate est donc retrouvé dans le NO synthétisé par la souche TL223.

On a, en outre observé que lorsque la souche TL223 est cultivée sur K¹⁵NO₃, le profil du pic de masse 31 (¹⁵N¹⁶O) augmente très fortement par rapport au NO analysé sur un milieu YEL contenant du nitrate non marqué, ce qui permet de confirmer cette situation.

De plus, l'addition de 1 mM de KNO₃ ou de KNO₂ non marqué entraîne une production de NO avec un rapport isotopique de 0,75 % (48 heures d'incubation - figure 8C), ce qui est très proche des valeurs du rapport isotopique naturel du NO (environ 0,4 %).

Ces dernières observations confirment de manière très claire que le gaz analysé en spectrométrie de masse était bien du NO.

Ces essais ont donc permis de constater que la souche TL223 est capable de synthétiser du NO directement à partir de nitrite ou en présence de nitrate après réduction de celui-ci en nitrite.

Sur la base des valeurs du rapport isotopique obtenues sur milieu YEL additionné de 1 mM de K¹⁵NO₃ marqué à 50 %, il est possible de déduire le taux de conversion du nitrate en nitrite : ainsi, environ 20% du K¹⁵NO₃ apporté est transformé en monoxyde d'azote par la souche TL223.

Le nitrate, initialement présent dans le milieu YEL stérile, explique la production de NO observée chez TL223 (de l'ordre de 2 µg de NO/ml après 72 heures d'incubation - figure 7).

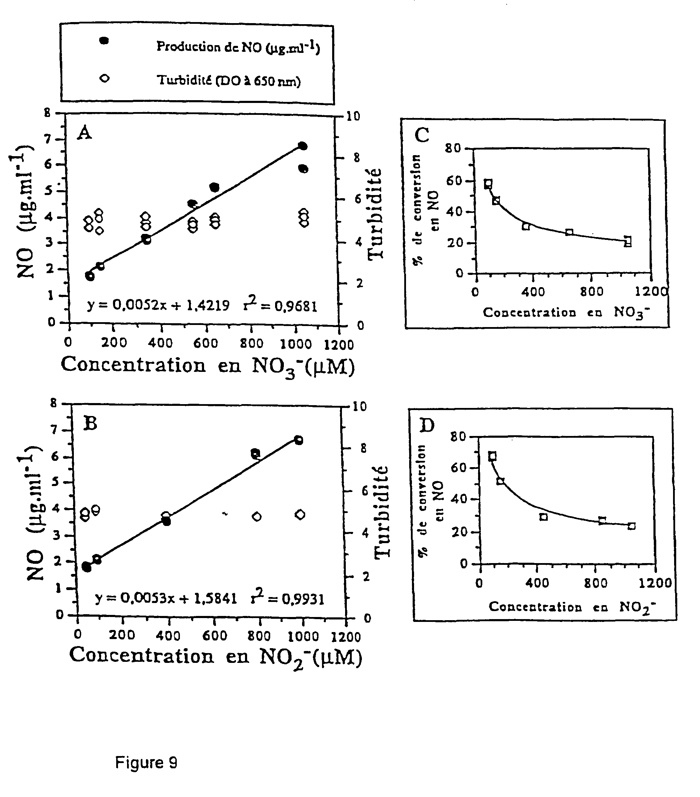

Les variations de la production de NO en fonction de la concentration en nitrite ou en nitrate sont représentées sur la figure 9. Plus précisément :
- la figure 9A représente les variations de la production de NO et l'évolution de la turbidité en fonction de la concentration initiale en nitrate, chez la souche TL223 cultivée sur milieu YEL après 72 heures d'incubation,
- la figure 9B représente les variations de la production de NO et l'évolution de la turbidité en fonction de la concentration initiale en nitrite, chez la souche TL223 cultivée sur milieu YEL après 72 heures d'incubation,
- la figure 9C représente les variations du taux de conversion du nitrate en NO, en fonction de la concentration initiale en nitrate,
- la figure 9D représente les variations du taux de conversion du nitrite en NO, en fonction de la concentration initiale en nitrite.

Sur ces figures, les concentrations en nitrate ont été corrigées en tenant compte de la présence d'environ 50 µM de nitrate dans le milieu YEL seul et sont les suivantes : 100, 150, 350, 550, 650 et 1050 µM.

Les concentrations en nitrite sont les suivantes : 50, 100, 400, 800 et 1000 µM.

Ces figures montrent que pour les gammes choisies, la production de monoxyde d'azote par la souche TL223 est proportionnelle à la concentration initiale en nitrate (figure 9A) ou en nitrite (figure 9B) du milieu YEL. Cette relation est linéaire.

Dans les deux cas, aucune phase de plateau n'a été observée, ce qui laisse supposer que les concentrations en nitrate ou nitrite utilisées ne permettent pas d'obtenir le niveau maximum d'accumulation de NO par la bactérie propionique TL223.

Il est également à noter que la présence de fortes concentrations en nitrate ou en nitrite n'affecte pas la croissance bactérienne vu que les valeurs de turbidité à 72 heures sont très proches pour toutes les concentrations qui ont été testées.

Il convient, par ailleurs, de souligner que les droites obtenues sur les figures 9A et 9B sont superposables, ce qui permet de prouver que le NO produit provient directement du nitrite ou du nitrate via la réduction de celui-ci en nitrite.

De plus, ces résultats montrent que chez la souche TL223, l'étape de réduction du nitrate en nitrite n'est pas limitante pour les concentrations en nitrate choisies dans cette expérimentation.

Il est également important de souligner que le taux de conversion du NO₃ (figure 9C) et du NO₂ (figure 9D) évolue en fonction de la quantité de substrat disponible, passant de 20 à 60 % lorsque la concentration en NO₃ ou NO₂ passe de 1000 à 100 µM.

Ceci suggère que la production de NO est fortement régulée et qu'elle est prioritaire par rapport à l'utilisation de l'azote nitrique pour les synthèses azotées.

A partir des conclusions susmentionnées, on a étudié la production de NO chez 12 souches de bactéries propioniques de deux espèces différentes. Les résultats de ces essais sont résumés ci-dessous :

### 6 - Etude de 1a production de NO chez différentes souches propioniques

Conformément à cet essai, les souches de bactéries propioniques retenues ont été les suivantes :
- *P.freudenreichii* :: LS410, LS2501, LS2502, ITG 23, CNRZ89, CNRZ277, CNRZ81.
- *P.acidipropionici* :: TL223, NCDO1072, PR75, CNRZ80, CNRZ86, CNRZ287.

Il est à noter que les souches CNRZ80, CNRZ81, CNRZ86, CNRZ89, CNRZ277 et CNRZ287 appartiennent à la collection publique INRA-CNRZ tandis que la souche NCDO1072 appartient à la collection britannique National Collection of Dairy Organisms ; les autres souches appartiennent quant à elles à des collections privées.

Après ensemencement sur milieu YEL en présence de 550 µM de nitrate, les cultures bactériennes ont immédiatement été réparties par 5 ml dans des tubes étanches et flushées à l'hélium (conditions anaérobies). Ensuite, les souches propioniques ont été soumises aux conditions expérimentales suivantes :
- incubation à 30°C pendant 24, 48 et 72 heures,
- 3 répétitions,
- accumulation du NO dans l'atmosphère et détermination du rapport isotopique par spectrométrie de masse,
- estimation de la fermentation de chaque culture mesurée par lecture de l'absorbance à 650 nm,
- dosage du nitrate et du nitrite après récupération des milieux bactériens, centrifugation et mesure par kit Boerhinger.

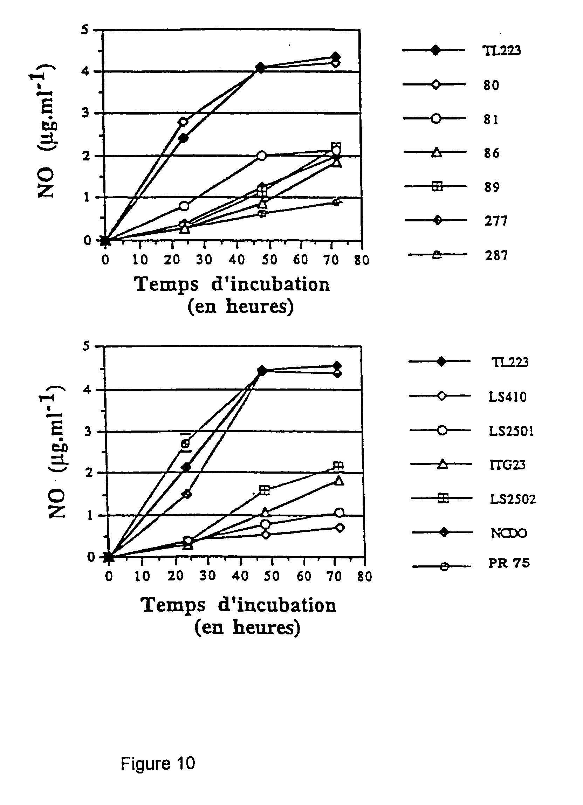

La figure 10 représente les variations de l'accumulation de NO en fonction du temps chez la souche TL223 et les 12 autres souches de bactéries propioniques, cultivées sur un milieu YEL en présence de 550 µM de nitrate. La souche TL223 a été représentée dans chaque cas dans un but de comparaison.

Les barres verticales représentent ± l'écart type de la moyenne pour n=3 lorsqu'elles sont plus grandes que le symbole.

Cette figure montre l'existence de fortes divergences entre les souches propioniques.

De manière globale, en comparant les niveaux de production de NO après 72 heures d'incubation, on peut classer les souches en trois catégories :
- souches capables de produire de 4 à 4,5 µg NO/ml : TL223, CNRZ80, NCDO1072 et PR75. Le rapport isotopique du NO produit par ces souches est compris entre 2 et 2,5 % (T=72 h),
- souches capables de produire environ 2 µg NO/ml : CNRZ81, CNRZ86, CNRZ89, CNRZ277, LS2502, ITG23. Le rapport isotopique du NO produit par ces souches se situe entre 4 et 5,5 % (T=72 h),
- souches produisant moins de 1 µg NO/ml : LS410, LS2501 et CNRZ287. Malgré la présence de 550 µM de nitrate dans le milieu de culture, ces 3 souches n'ont produit que de très faibles quantités de NO et le rapport isotopique était de l'ordre de 10 à 13 % (T=72 h). Ces valeurs suggèrent que les pics de masses 30 et 31 détectés chez ces bactéries pourraient ne pas correspondre à du monoxyde d'azote.

Il est à noter que chez les souches appartenant aux deux premières catégories, le NO produit ne diminue pas en fin de croissance et qu'il ne semble donc pas réutilisé par les bactéries propioniques : il y a donc accumulation du NO.

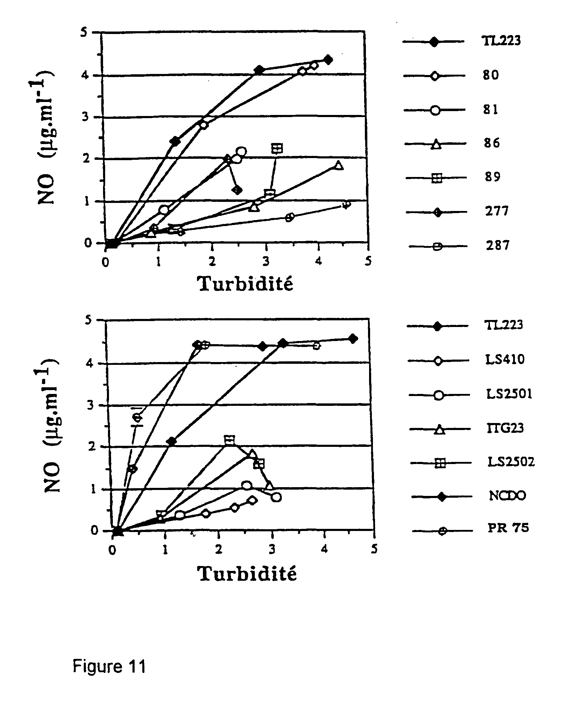

La figure 11 représente les variations de la production de NO en fonction de la turbidité chez les 13 souches de bactéries propioniques susmentionnées, cultivées sur milieu YEL en présence de 550 µM de nitrate. la souche TL223 a été représentée dans chaque cas dans un but comparatif.

Les barres verticales représentent ± l'écart type de la moyenne pour n=3, lorsqu'elles sont plus grandes que le symbole.

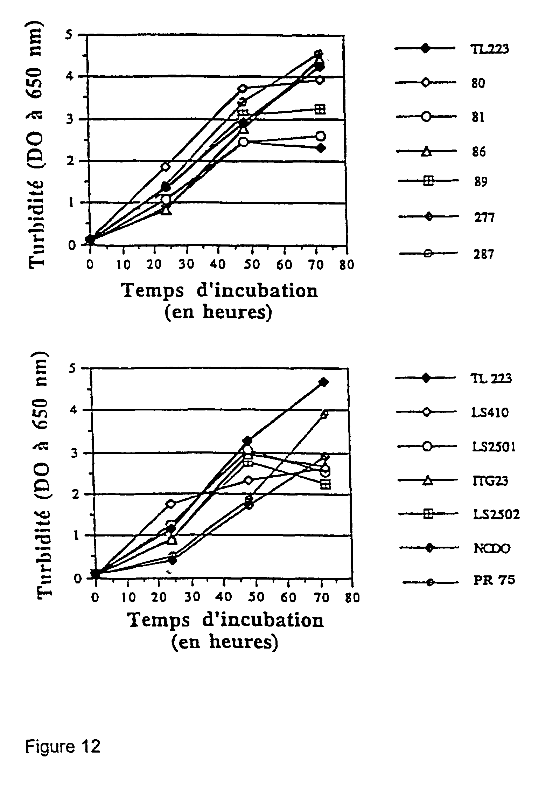

La figure 12 représente les variations de la turbidité (DO à 650 nm) en fonction du temps chez les 13 souches de bactéries propioniques analysées, cultivées sur milieu YEL en présence de 550 µM de nitrate.

La souche TL223 est représentée, dans chaque cas, dans un but comparatif.

Les barres verticales représentent l'écart type de la moyenne pour n=3, lorsqu'elles sont plus grandes que le symbole.

Les souches produisant le plus de NO (TL223, CNR280, NCDO1072 et PR75) atteignent toutes des valeurs de turbidité élevées (4 à 5 unités DO après 72 heures d'incubation.

Parmi ces souches, il est à noter que les bactéries NCDO1072 et PR75 croissent moins vite que TL223, mais sont capables d'accumuler très rapidement de fortes concentrations en NO.

Ainsi, PR75 produit 2,8 µg de NO/ml pour une turbidité de 0,5 unités DO. Le maximum de production de NO est enregistré pour des valeurs de turbidité d'environ 1,5 unité DO pour NCDO1072 et PR75, contre 3,4 unités DO pour TL223.

Pour « affiner » ces résultats, on a analysé l'évolution des concentrations en NO, nitrate et nitrite des milieux de culture des 13 souches de bactéries propioniques étudiées en fonction du temps d'incubation.

Les résultats obtenus sont rassemblés dans le tableau ci-dessous dans lequel les concentrations sont exprimées en µM. Pour chaque souche, les mesures ont été effectuées sur un tube, après élimination des bactéries par centrifugation.

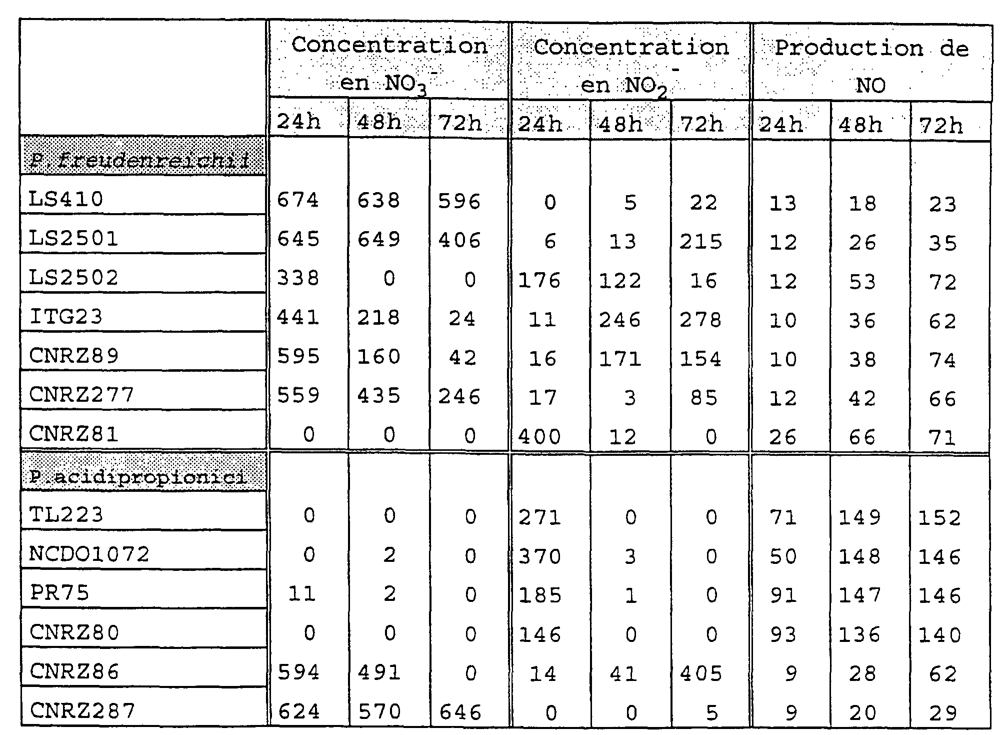

Ce tableau permet de faire les constatations suivantes :
- les souches produisant 4 µg NO/ml sont capables de réduire entièrement le nitrate disponible (soit 550 µM) après 24 heures d'incubation. De plus, elles peuvent réduire totalement le nitrite obtenu au cours des 48 premières heures d'incubation
- les souches LS410 et CNRZ287, très faibles productrices de NO, ne sont pas capables d'absorber significativement le nitrate présent dans le milieu.
- chez les souches présentant une accumulation intermédiaire de NO, on peut observer des évolutions des teneurs en nitrate et en nitrite très différentes traduisant des vitesses d'absorption du NO₃⁻ et/ou de réduction du NO₃⁻ et du NO₂⁻ très différentes. Ainsi, la souche CNRZ81 est capable de réduire la totalité du nitrate après 24 heures d'incubation. Après 48 heures, CNRZ81 réalise également la réduction de l'ensemble du NO₂⁻ obtenu par réduction du NO₃⁻. A l'opposé, CNRZ277 possède encore 246 µM de NO₃⁻ et 85 µM de NO₂⁻ après 72 heures d'incubation.

Les essais résumés ci-dessus ont révélé que certaines souches propioniques sont capables de réduire le nitrate du milieu de culture et de fournir ainsi le nitrite nécessaire à la synthèse de NO.

Il est à noter que les souches propioniques qui produisent le plus de NO (TL223, CNRZ80, NCDO1072 et PR75) appartiennent toutes à l'espèce *P.acidipropionici* et possèdent, en outre, toutes une activité nitrate réductase.

A l'inverse, les souches produisant apparemment moins, voire pas du tout, de NO (limite de détection du spectromètre de masse) sont également des bactéries qui ne possèdent pas d'activité nitrate réductase connue (LS410, LS2501 et CNRZ287).

Pour certaines souches propioniques, il semble que la cinétique de production de NO ne soit pas directement liée à l'activité nitrate réductase.

Compte tenu de ces résultats, l'invention se rapporte également à une composition diététique ou médicamenteuse absorbable, caractérisée en ce qu'elle est constituée par une préparation renfermant une quantité importante de préférence plus de 10⁹ cellules/g, de souches de bactéries propioniques sélectionnées en fonction de leur capacité à dégager et/ou à accumuler du monoxyde d'azote à raison d'au moins 1 µg/ml de milieu YEL contenant 550 µM de nitrate.

Selon une autre caractéristique de l'invention, cette composition renferme des bactéries propioniques appartenant à au moins l'une des souches TL223, CNRZ80, CNRZ86 et NCDO1072 de l'espèce *P.acidipropionici.*

Parmi ces souches, la souche TL223 s'est révélée particulièrement avantageuse.

Il est également à noter que la souches CNRZ80 présente un intérêt tout particulier d'un point de vue « productivité » vu qu'elle est capable d'accumuler une concentration importante de NO, ce très rapidement (pour des valeurs de turbidité relativement faibles).

Selon une autre caractéristique de l'invention, cette composition renferme des bactéries propioniques appartenant à au moins l'une des souches ITG23, CNRZ81, CNRZ89, CNRZ277 et LS2502 de l'espèce *P.freudenreichii.*

Il est à noter que, conformément à une autre caractéristique de l'invention, la composition peut également renfermer d'autres bactéries telles que des bifidobactéries et/ou des bactéries lactiques.

Pour compléter les résultats obtenus ci-dessus, on a effectué des tests sur deux types de bactéries non propioniques : E.coli et Lactobacillus farciminis connues pour leur aptitude à réduire les nitrites.

Les résultats de ces tests sont résumés ci-dessous :

### 7 - Etude de la production de NO par les souches E.coli et L.farciminis

Ces tests complémentaires ont principalement été effectués compte tenu de l'existence de la publication « Heme-dependent and heme-independent nitrite reduction by lactic acid bacteria results in different N-containing products » Gudrun Wolf, Elke K. Arendt, Ute Pfähler and Walter P. Hammes - International Journal of Food Microbiology, 10 (1990) 323-330) qui mentionne que certaines bactéries lactiques (L.farciminis) sont capables de produire du monoxyde d'azote à partir de nitrite.

Des expériences préliminaires ont montré qu'après 5 h 30 de croissance de la souche L.farciminis dans du MRS additionné de 1 mM de nitrate, on ne détectait plus de nitrate ni de nitrite dans le milieu de culture.

La même constatation a été faite en ce qui concerne la souche E.coli après 7 h 30 de croissance sur milieu BHI additionné de 1 mM de nitrate.

Il est connu, qu'au cours de sa croissance, la souche L.farciminis acidifie le milieu MRS (environ pH 5 après 5-6 heures de culture).

Or, on a pu constater, à partir d'essais réalisés sur milieu YEL, que les nitrites se transforment en NO en milieu acide.

Ces essais ont été réalisés dans les conditions expérimentales suivantes :
- acidification du milieu YEL par de l'HCl,
- apport de nitrite à une concentration de 400 µM,
- autoclavage des milieux,
- trois répétitions.

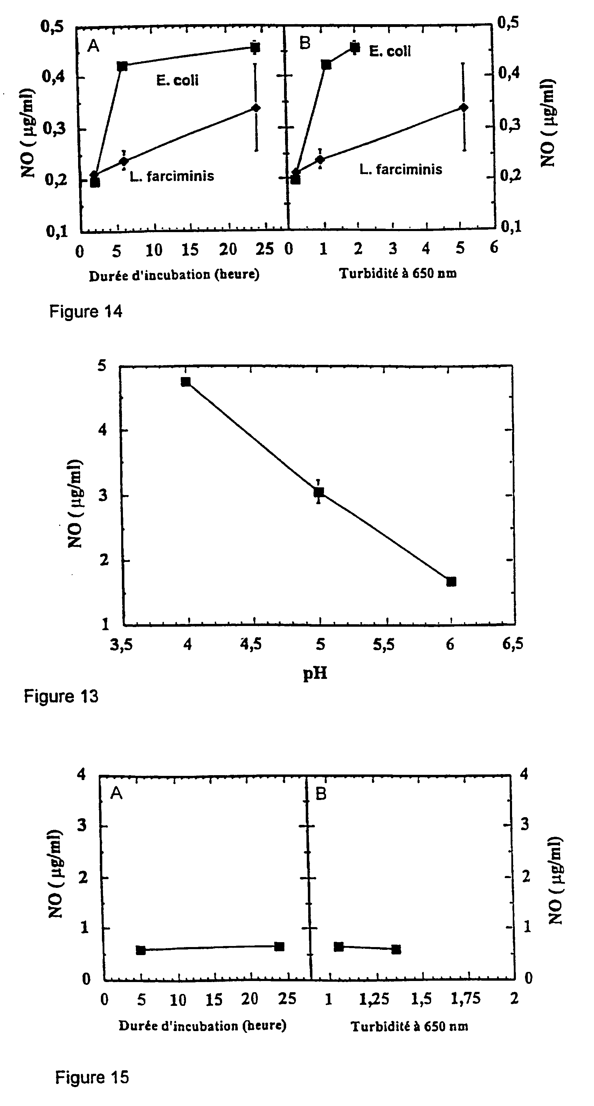

Les résultats obtenus sont rassemblés sur la figure 13 qui représente les variations en fonction du pH de la production de NO dans le milieu YEL additionné de nitrites après incubation à 37°C pendant 24 heures.

Cette figure est de nature à prouver qu'il existe une production notable de NO à partir du nitrite du milieu lorsque celui-ci est acide, cette production augmentant lorsque le pH diminue.

Par suite, on a effectué des tests comparatifs de la production de NO par L.farciminis et par E.coli qui est réputée non productrice de NO (Brittain T, Blackmore R, Greenwood C & Thomson AJ (1992) - Bacterial nitrite - reducing enzymes - Eur. J. Bio. Chem., 209, 793-802).

Ces tests ont été réalisés dans les conditions suivantes :
- incubation à 37°C sur milieu BHI (E.coli) ou MRS (L.farciminis),
- apport de nitrate dans le milieu à une concentration de 1 mM,
- flushage de l'atmosphère de chaque tube par de l'hélium pendant 100 secondes,
- trois répétitions,
- mesure de la turbidité en fin d'incubation.

Ces essais ont permis d'obtenir les résultats rapportés sur la figure 14.

Plus précisément :
- la figure 14A représente les variations de la production de NO en fonction du temps d'incubation,
- la figure 14B représente les variations de la production de NO en fonction de la turbidité du milieu.

Il est à noter que les valeurs obtenues pour la production de NO sont toutes nettement inférieures au seuil de 1 µg/ml qui a été considéré ci-dessus comme significatif : il en résulte qu'il n'y a pas accumulation de NO dans les tubes de culture.

Ces résultats indiquent donc que l'absence de nitrate et de nitrite observée dans l'expérience préliminaire après respectivement 5 h 30 (L.farciminis) et 7 h 30 (E.coli) n'est pas compensée par une accumulation de NO qui pourrait être soit d'origine chimique (liée à l'acidification du milieu) soit d'origine bactérienne.

Ces résultats ont été, dans le cas de la souche L.farciminis, confirmés par des tests effectués sur des bactéries se trouvant sous forme de resting cells à pH régulé à 6,5 par un tampon phosphate contenant du lactate dans les conditions opératoires suivantes :
- incubation à 37°C,
- apport de nitrite à une concentration de 400 µM,
- flushage de l'atmosphère de chaque tube par de l'hélium pendant 100 secondes,
- trois répétitions,
- mesure de la turbidité en fin d'incubation.

Cette analyse a permis d'obtenir les résultats rapportés sur la figure 15 :
- la figure 15A représente les variations de la production de NO en fonction du temps d'incubation,
- la figure 15B représente les variations de la production de NO en fonction de la turbidité.

Ces résultats confirment ceux obtenus précédemment, à savoir que les quantités de NO produites sont trop faibles pour être significatives et donc que la souche L.farciminis n'est pas susceptible d'accumuler le monoxyde d'azote. Il est toutefois possible que cette souche produise du monoxyde d'azote en début de croissance, mais que le NO éventuellement produit soit réutilisé par la bactérie.

Des essais complémentaires ont été réalisés sur les souches TL223 et CNRZ80 sous forme de resting cells après incubation à 30 et également à 37°C.

### 8 - Evolution de la production de NO par des bactéries propioniques sous forme de resting cells

Cette expérimentation a été effectuée dans les conditions suivantes :
- resting cells suspendues dans un tampon phosphate contenant du lactate à pH 6,5,
- incubation à 30°C ou à 37°C,
- apport de nitrite à une concentration de 400 µM,
- flushage de l'atmosphère de chaque tube par de l'hélium pendant 100 secondes,
- trois répétitions,
- mesure de la turbidité en fin d'incubation.

Il est à noter que lors des tests relatifs à l'incubation à 30°C, on a examiné, outre les souches TL223 et CNRZ80, la souche CNRZ81 avec une concentration bactérienne doublée.

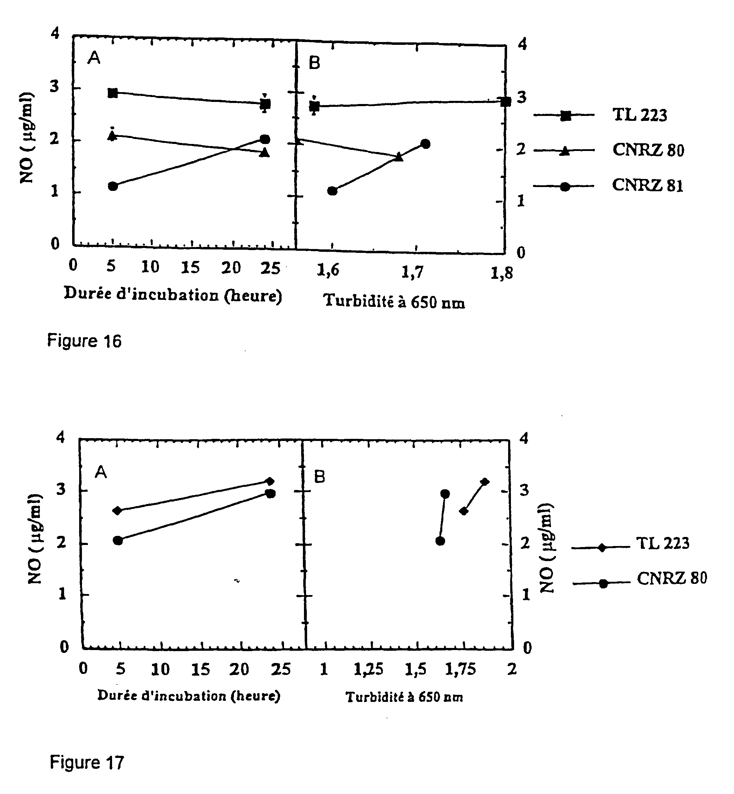

Cette expérimentation a permis d'obtenir les résultats rapportés sur les figures 16 et 17. Plus précisément :
- la figure 16A représente les variations de la production de NO par des resting cells à 30°C en fonction de la durée d'incubation,
- la figure 16B représente les variations de la production de NO par des resting cells à 30°C en fonction de la turbidité du milieu,
- la figure 17A représente les variations de la production de NO par des resting cells à 37°C en fonction de la durée d'incubation,
- la figure 17B représente les variations de la production de NO par des resting cells à 37°C en fonction de la turbidité du milieu.

Ces résultats sont de nature à prouver qu'il existe une production conséquente de NO par des resting cells non seulement dans le cas des deux souches de l'espèce *P.acidipropionici* (TL223 et CNRZ80) mais également dans le cas de la souche de l'espèce *P.freundenreichii* (CNRZ81). La souche TL223 est la plus productive.

Globalement, à des concentrations identiques de bactéries, la production de NO par des resting cells est du même ordre que celle observée dans le cas de bactéries cultivées sur milieu YEL.

La production de NO par des resting cells intervient essentiellement lors des cinq premières heures d'incubation ; au-delà de cette période, la production est faible.

On a ainsi pu observer qu'à 37°C, la production de NO est identique (TL223) ou légèrement supérieure (CNRZ80) à celle obtenue à 30°C.

Les avantages associés à l'ingestion de bactéries propioniques ont, en outre, été vérifiés par des investigations réalisées in vivo chez l'homme sain.

### 9 - Etude de l'effet de l'ingestion de bactéries propioniques sur le transit intestinal chez l'homme sain

Cette étude a été réalisée en milieu hospitalier au CHU de Caen sur une série de 19 sujets masculins volontaires sains.

Au début de ce test, on a fait absorber quotidiennement à chaque volontaire 10 marqueurs radio opaques, ce pendant 8 jours consécutifs, conformément au protocole décrit dans les publications *Arhan P, Devroede G, Jehannin B et coll. Dis Colon Rectum 1981 ; 24:625-9.* et *Bouchoucha M, Devroede G. Arhan P et coll. Dis Colon Rectum 1992 ; 35:773-82.*

Selon ce protocole, l'étude du transit est effectuée par comptage des marqueurs radio opaques ingérés dans les différentes aires de la cavité abdominale répartis sur un cliché d'abdomen de face. Ces aires (côlon droit, côlon gauche et rectosigmoide) sont définies par des lignes fictives joignant la 5^{ième} vertèbre lombaire au contour de la cavité pelvienne. Le temps de transit est calculé selon la formule T = 1/N . n . △t ; N étant égal à 10 marqueurs, n représentant le nombre de marqueurs comptés dans une région et △t étant égal à 24 heures.

Le jour suivant cette ingestion, c'est-à-dire le 9^{ième} jour, on a fait subir aux volontaires une radiographie de l'abdomen de face sans préparation.

A partir du jour suivant, c'est-à-dire du 10^{ième} jour, on a fait ingérer quotidiennement à chaque volontaire, ce pendant 2 semaines, une gélule contenant 5 10¹⁰ bactéries propioniques issues d'une banque de souches utilisées dans l'industrie fromagère, donc parfaitement inoffensives pour l'homme.

Une seconde étude du temps de transit similaire à la première a été effectuée durant la seconde semaine d'ingestion des propioniques, c'est-à-dire du 17^{ième} au 26^{ième} jour.

Cette étude a révélé un ralentissement significatif du temps de transit du côlon gauche (p < 0,05 conformément au test statistique Wilcoxon Matched-Paired Signed-Ranks Test.) ; les temps de transit du colon droit et du rectosigmoïde n'ont pas été significativement modifiés par l'ingestion des propioniques.

Cette étude a donc permis de prouver que l'ingestion de bactéries propioniques a une influence sur la motricité intestinale ; on peut supposer que ces résultats sont liés à la synthèse de monoxyde d'azote par les bactéries propioniques.

## Revendications

1. Utilisation de bactéries propioniques pour l'obtention d'une composition d'alimentation courante ou d'une composition diététique ou médicamenteuse absorbable susceptible de dégager des quantités physiologiquement significatives de monoxyde d'azote dans le tube digestif humain ou animal.

2. Utilisation selon la revendication 1,
**caractérisée en ce que**
la composition est constituée par une préparation déshydratée.

3. Utilisation selon la revendication 2,
**caractérisée en ce que**
la composition se présente sous forme de fractions individuelles renfermant la dose de bactéries devant être absorbée régulièrement.

4. Utilisation selon la revendication 3,
**caractérisée en ce que**
chaque fraction individuelle renferme plus de 10⁹ bactéries.

5. Utilisation selon la revendication 1,
**caractérisée en ce que**
la composition est constituée par une préparation liquide fermentée ou non.

6. Utilisation selon la revendication 1,
**caractérisée en ce que**
la composition est une préparation élaborée, les bactéries propioniques étant ajoutées ou incorporées dans des aliments tels que des fromages ou des fibres alimentaires.

7. Composition diététique ou médicamenteuse absorbable,
**caractérisée en ce qu'**
elle est constituée par une préparation renfermant une quantité importante de préférence plus de 10⁹ cellules/g de souches de bactéries propioniques sélectionnées en fonction de leur capacité à dégager et/ou à accumuler du monoxyde d'azote à raison d'au moins 1 µg/ml de milieu YEL contenant 550 µM de nitrate.

8. Composition selon la revendication 7,
**caractérisée en ce qu'**
elle renferme des bactéries propioniques appartenant à au moins l'une des souches TL223, CNRZ80, CNRZ86 et NCDO1072 de l'espèce *P.acidipropionici*.

9. Composition selon la revendication 8,
**caractérisée en ce qu'**
elle renferme des bactéries propioniques appartenant à la souche TL223 de l'espèce *P.acidipropionici*.

10. Composition selon la revendication 8,
**caractérisée en ce qu'**
elle renferme des bactéries propioniques appartenant à la souche CNRZ80.

11. Composition selon la revendication 7,
**caractérisée en ce qu'**
elle renferme des bactéries propioniques appartenant à au moins l'une des souches ITG23, CNRZ81, CNRZ89, CNRZ277 et LS2502 de l'espèce *P.freudenreichii.*

12. Composition selon l'une quelconque des revendications 7 à 11,
**caractérisée en ce qu'**
elle renferme en outre d'autres bactéries telles que des bifidobactéries et/ou des bactéries lactiques

## Patentansprüche

1. Verwendung von Propionsäurebakterien zur Gewinnung einer gewöhnlichen Nahrungsmittel-Zusammensetzung oder einer diätetischen oder medikamentösen Zusammensetzung, die absorbierbar und fähig ist, physiologisch signifikante Mengen von Stickstoffoxid im menschlichen oder tierischen Verdauungstrakt zu produzieren.

2. Verwendung nach Anspruch 1, **dadurch gekennzeichnet, daß** die Zusammensetzung von einem dehydrisierten Präparat gebildet ist.

3. Verwendung nach Anspruch 2, **dadurch gekennzeichnet, daß** die Zusammensetzung in Form einzelner Fraktionen vorliegt, die die Bakteriendosis, bevor sie regulär absorbiert werden, enthalten.

4. Verwendung nach Anspruch 3, **dadurch gekennzeichnet, daß** jede einzelne Fraktion mehr als 10⁹ Bakterien enthält.

5. Verwendung nach Anspruch 1, **dadurch gekennzeichnet, daß** die Zusammensetzung von einem Flüssigpräparat gebildet ist, das fermentiert ist oder nicht.

6. Verwendung nach Anspruch 1, **dadurch gekennzeichnet, daß** die Zusammensetzung eine fertige Zubereitung ist, wobei die Propionsäurebakterien Nahrungsmitteln wie Käse oder Nahrungsmittelfasern zugesetzt oder in diese eingearbeitet sind.

7. Absorbierbare diätetische oder medikamentöse Zusammensetzung, **dadurch gekennzeichnet, daß** sie von einem Präparat gebildet ist, das eine erhebliche Menge von vorzugsweise mehr als 10⁹ Zellen/g von Stämmen von Propionsäurebakterien enthält, ausgewählt in Abhängigkeit von ihrer Fähigkeit, Stickstoffoxid zu zumindest 1µg/ml YEL-Milieu mit 550 µM Nitrat zu produzieren und/oder anzusammeln.

8. Zusammensetzung nach Anspruch 7, **dadurch gekennzeichnet, daß** sie Propionsäurebakterien enthält, die zu zumindest einem der Stämme TL223, CNRZ80, CNRZ86 und NCDO1072 der Spezies *P.acidipropionici* gehören.

9. Zusammensetzung nach Anspruch 8, **dadurch gekennzeichnet, daß** sie Propionsäurebakterien enthält, die zum Stamm TL223 der Spezies *P.acidipropionici* gehören.

10. Zusammensetzung nach Anspruch 8, **dadurch gekennzeichnet, daß** sie Propionsäurebakterien enthält, die zum Stamm CNRZ80 gehören.

11. Zusammensetzung nach Anspruch 7, **dadurch gekennzeichnet, daß** sie Propionsäurebakterien enthält, die zu zumindest einem der Stämme ITG23, CNRZ81, CNRZ 89, CNRZ277 und LS2502 der Spezies *P. freudenreichii* gehören.

12. Zusammensetzung nach einem der Ansprüche 7 bis 11, **dadurch gekennzeichnet, daß** sie außerdem andere Bakterien wie Bifidusbakterien und/oder Laktobakterien enthält.

## Claims

1. Use of propionic bacteria to obtain an absorbable common food composition or a dietetic or medicinal composition capable of releasing physiologically significant quantities of nitric oxide in the human or animal alimentary canal.

2. Use as claimed in claim 1,
**characterised in that**
the composition is a dehydrated preparation.

3. Use as claimed in claim 2,
**characterised in that**
the composition is in the form of individual fractions containing the dose of bacteria to be regularly absorbed.

4. Use as claimed in claim 3,
**characterised in that**
each individual fraction contains more than 10⁹ bacteria.

5. Use as claimed in claim 1,
**characterised in that**
the composition is a liquid preparation, fermented or not.

6. Use as claimed in claim 1,
**characterised in that**
the composition is a ready-made preparation, the propionic bacteria being added or incorporated in foodstuffs such as cheese or dietary fibre.

7. Absorbable dietetic or medicinal composition,
**characterised in that**
it is a preparation containing a large quantity, preferably in excess of 10⁹ cells/g, of propionic bacterial strains selected on the basis of their capacity to release and/or accumulate nitric oxide to the extent of at least 1 µg/ml of YEL medium containing 550 µM of nitrate.

8. Composition as claimed in claim 7,
**characterised in that**
it contains propionic bacteria belonging to at least one of the TL223, CNRZ80, CNRZ86 and NCDO1072 strains of the *P.acidipropionici* species.

9. Composition as claimed in claim 8,
**characterised in that**
it contains propionic bacteria belonging to the TL223 strain of the *P.acidipropionici* species.

10. Composition as claimed in claim 8,
**characterised in that**
it contains propionic bacteria belonging to the CNRZ80 strain.

11. Composition as claimed in claim 7,
**characterised in that**
it contains propionic bacteria belonging to at least one of the ITG23, CNRZ81,CNRZ89, CNRZ277 and LS2502 strains of the *P.freudenreichii* species.

12. Composition as claimed in any one of claims 7 to 11,
**characterised in that**
it additionally contains other bacteria, such as bifidobacteria and/or lactic bacteria.
